# EUROPÄISCHE PATENTSCHRIFT

(11) **EP 1 712 255 B1**
(45) Veröffentlichungstag und Bekanntmachung des Hinweises auf die Patenterteilung: **12.10.2016**
(21) Anmeldenummer: 06112481.4
(22) Anmeldetag: 11.04.2006
(51) Int. Cl.: A61K 8/35, A61Q 5/06, A61K 8/81

(54) **Kosmetische Zubereitungen enthaltend eine Kombination aus einem Copolymer, mindestens einem nichtionischen Polymer und Cetrimonium Chlorid.**
Cosmetic compositions comprising a combination of a copolymer, at least one noniionic polymer and cetrimonium chloride.
Compositions cosmétiques comprenant une combinaison d'un copolymère, d'au moins un polymère non-ionique et chlorure de cétrimonium.

(30) Priorität: 14.04.2005 DE 102005017465
(43) Veröffentlichungstag der Anmeldung: 18.10.2006
(73) Patentinhaber: Beiersdorf Aktiengesellschaft, 20253 Hamburg (DE)
(72) Erfinder: Franck, Kerstin, 22529, Hamburg (DE); Argembeaux, Horst, 21465, Wentorf (DE); Saß, Viola, 25488, Holm (DE); Detert, Marion, 22455, Hamburg (DE)

(56) Entgegenhaltungen:
- WO-A-2005/123014
- ANONYMOUS: "WÃ ssrige Zubereitungen enthaltend wenigstens ein wasserlÃsliches oder wasserdispergierbares Copolymer mit kationogenen Gruppen" IP.COM JOURNAL, IP.COM INC., WEST HENRIETTA, NY, US, 7. März 2005 (2005-03-07), XP013023623 ISSN: 1533-0001

## Beschreibung

Die vorliegende Erfindung betrifft eine kosmetische Zubereitung enthaltend eine Kombination aus einem Copolymer gebildet aus den Monomeren N-Vinylpyrrolidon (VP). N-Vinylimidazol (VI), Methacrylamid (MAM) und quaternisiertem N-Vinylimidazol (QVI), mindestens einem nichtionischen Polymer und Cetrimoniumchlorid sowie deren Verwendung als Haarfestiger.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl Styling) vielseitiger Frisuren verwendet man Haarfestiger, meist in Form von Schaumfestigern oder Haarsprays. Beide haarkosmetischen Mittel sind in Ihrer Zusammensetzung ähnlich unterscheiden sich aber in ihrer Aufgabe und Anwendung. Schaumfestiger (auch Schaumhaarfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die Rezepturen für derartige Produkte sind vielfältig. Die Bestandteile müssen jedoch in ethanolischem, wässrig-ethanolischem oder wässrigem Medium verträglich sein.

Üblicherweise bestehen diese Mittel zur Festigung der Haare (Haarfestiger) aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation einen transparenten farblosen Film. Es werden eine Reihe von Anforderungen an diese Filme gestellt: sie sollen klar, glänzend, feuchtigkeitsunempfindlich, festigend, lang anhaltend, flexibel, nicht klebend sein, den natürlichen Griff des Haares nicht beeinträchtigen und sich auch wieder leicht durch handelsübliche Haarwaschmittel auswaschen lassen. Schaumfestiger besitzen in der Gruppe der Stylingprodukte ein Sonderstellung. Nicht nur die Filmeigenschaften (Halt, Flexibilität...) stehen im Vordergrund, sondern auch die Pflegeleistung am Haar (Kämmbarkeit, gepflegter Griff) und die Schaumcharakteristik (feinblasig, cremig, stabil) haben einen beträchtlichen Einfluss auf die Produktperformance.

Kationische Filmbildner weisen vor allem pflegende Eigenschaften auf: das Haar lässt sich gut kämmen und fühlt sich geschmeidig und gepflegt an. Insbesondere für Schaumfestiger hat die Kämmbarkeit und der Griff der nassen und trockenen Haare eine entscheidende Bedeutung. Als entscheidender Nachteil dieser Rohstoffe ist ihre geringe Festigungsleistung zu nennen.

Die Verwendung von Mischungen kationischer Filmbildner zur Produktoptimierung von haarkosmetischen Zubereitungen ist dem Fachmann an sich bekannt.

Eine Verbesserung in Bezug auf die Festigungsleistung wird bei Styling-Formulierungen auf Basis kationischer Polymere üblicherweise durch die Kombination mit nichtionischen Filmbildnern erreicht.

Der Einsatz dieser nichtionischen Polymere führt jedoch in der Regel zu einer deutlichen Abnahme der Pflegeeigenschaften. Unter Pflegeeigenschaften versteht der Fachmann die Wz-rk-02180

Fähigkeit des Produktes die Haare zu glätten, was zu einer Verbesserung der Kämmbarkeit der nassen und trockenen Haare führt. Produkte mit guten Pflegeeigenschaften erhöhen den natürlichen Glanz des Haares, vermindern die elektrostatische Aufladung und verleihen dem Haar einen angenehmen, geschmeidigen Griff (im nassen und trockenen Haar). Die Flexibilität des Polymerfilms wird verbessert. Gemessen werden die Pflegeeigenschaften in erster Linie durch die Bestimmung der Kämmkraft (nass oder trocken).

Es war daher die Aufgabe der vorliegenden Erfindung, die Nachteile des Standes der Technik zu beseitigen und eine kosmetische Zubereitung (insbesondere einen Schaumfestiger für das Haar) zu entwickeln, welche hohe Haarfestigungseigenschaften und hervorragende Pflegeeigenschaften (wie eine gute Kämmbarkeit und einen gepflegten Griff) aufweist.

Überraschend gelöst wird die Aufgabe durch eine kosmetische Zubereitung enthaltend eine Kombination aus
a) einem Copolymer gebildet aus den Monomeren N-Vinylpyrrolidon (VP), N-Vinylimidazol (VI), Methacrylamid (MAM) und quaternisiertem N-Vinylimidazol (QVI),
b) mindestens einem nichtionischen Polymer, wobei ein oder mehrere nichtionische Polymere gewählt werden aus der Gruppe der Homopolymere des Vinylpyrrolidons, des Vinylcaprolactams, aus der Gruppe der Copolymerisate aus Vinylpyrrolidon und Vinylacetat, aus der Gruppe der Polyvinylcaprolactame, Polyvinylamide, aus der Gruppe der Copolymerisate aus Vinylpyrrolidon und Dimethylaminoethylmethacrylat, aus der Gruppe der Terpolymere aus Vinylpyrrolidon, Vinylimidazol und Methacrylamid oder aus der Gruppe der Terpolymere aus Vinylpyrrolidon, Vinylimidazol und Vinylcaprolactam,
c) Cetyltrimethylammoniumchlorid (Cetrimonium chloride; CAS 112-02-7).

Die erfindungsgemäßen Zubereitungen führen zu einer deutlichen Verbesserung der Pflegeleistung unter gleichzeitiger Erhöhung der Festigungsleistung bei ihrer Anwendung als Haarfestiger.

Liegen die erfindungsgemäßen Zubereitungen in Form eines Schaumfestigers vor, so weist die Zubereitung einen besonders cremigen, feinblasigen Schaum auf der sich leicht und gleichmäßig im Haar verteilen lässt.

Das Haar lässt sich nach der Anwendung besonders leicht kämmen und weist einen gesunden, seidigen Glanz auf. Die Bildung von aus dem Haar herab rieselnden Filmplaken ist deutlich reduziert.

Zwar ist dem Fachmann der Einsatz von einem Copolymer gebildet aus den Monomeren N-Vinylpyrrolidon (VP), N-Vinylimidazol (VI), Methacrylamid (MAM) und quaternisiertem N-Vinylimidazol (QVI) in kosmetischen Zubereitungen an sich bekannt. So offenbart der "Technical Disclosure" "Wässrige Zubereitungen enthaltend wenigstens ein wasserlösliches oder wasserdispergierbares Copolymer mit kationogenen Gruppen", veröffentlicht in "IP com" Prior Art Database vom 16.3.2005 (link: http://ip.com/pubView/IPCOM000097478D) kosmetische Zubereitungen mit derartigen Polymeren. Doch konnte diese Veröffentlichung nicht den Weg zur vorliegenden Erfindung weisen, da keine Möglichkeiten zur Verbesserung der Halte- oder Pflegeleistung von haarkosmetischen Zubereitungen beschrieben wurden.

Es ist erfindungsgemäß vorteilhaft, wenn die kosmetische Zubereitung das Copolymer aus den Monomeren N-Vinylpyrrolidon (VP), N-Vinylimidazol (VI), Methacrylamid (MAM) und quaternisiertem N-Vinylimidazol (QVI) in einer Konzentration von 0,01 bis 10 Gewichts-%, und bevorzugt in einer Konzentration von 2,5 bis 7,5 Gewichts-% (tel quel), jeweils bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung nichtionische Polymere in einer Konzentration von 0,01 bis 20 Gewichts-% und bevorzugt von 4 bis 10 Gewichts-% (tel quel), jeweils bezogen auf das Gesamtgewicht der Zubereitung, enthält.

Vorteilhaft im Sinne der vorliegenden Erfindung ist es, wenn das Gewichtsverhältnis von Copolymer aus den Monomeren N-Vinylpyrrolidon (VP), N-Vinylimidazol (VI), Methacrylamid (MAM) und quaternisiertem N-Vinylimidazol (QVI) zu nichtionischen Polymeren von 5:1 bis 1:5 und bevorzugt von 1:1 bis 1:4 beträgt.

Eine erfindungsgemäß besonders bevorzugte Ausführungsform der vorliegenden Erfindung ist dadurch gekennzeichnet, dass das Copolymer aus N-Vinylpyrrolidon (VP), N-Vinylimidazol (VI), Methacrylamid (MAM) und quaternisiertem N-Vinylimidazol (QVI) die Monomere N-Vinylpyrrolidon (VP), N-Vinylimidazol (VI), Methacrylamid (MAM) und quaternisiertem N-Vinylimidazol (QVI) in einem Gewichtsverhältnis von 55:10:29:6 enthält. Dabei können die einzelnen Mengenbestandteile herstellungsbedingt schwanken. Abweichungen von 10 % sind als erfindungsgemäß anzusehen.

Es ist erfindungsgemäß besonders bevorzugt, wenn als nichtionisches Polymer ein Copolymerisat aus Vinylpyrrolidon und Vinylacetat (VP/VA-Copolymer)- eingesetzt wird. Beispielsweise können die folgenden nichtionischen VP/VA-Copolyere eingesetzt werden:
Luviskol VA 37 E, Luviskol VA 64 W, Luviskol VA 64 Powder, Luviskol VA 73 E oder Luviskol VA 73 W (Fa. BASF) oder PVP/VA S-630 PVP/VA E-735, PVPNA W-735, PVP/VA E-635, PVP/VA W-635, PVP/VA E-535 oder PVP/VA E-335 (Fa. ISP); INCI: VPNA Copolymer.

Die erfindungsgemäßen Zubereitungen können erfindungsgemäß vorteilhaft weitere kationische Polymere und/oder kationische Tenside in einer Gesamtkonzentration von 0,1 bis 5 Gewichts-%, bezogen auf das Gesamtgewicht der Zubereitung, enthalten.

Als erfindungsgemäß vorteilhafte kationische Polymere können Polyquaternium-4, Polyquaternium-6, Polyquaternium-7, Polyquaternium-10, Polyquaternium-11, Polyquaternium-16, Polyquaternium-22, Polyquaternium-44, Polyquaternium-46, Polyquaternium-55 und/oder quaternisierte Guarderivate eingesetzt werden.

Es ist erfindungsgemäß vorteilhaft, wenn die erfindungsgemäße Zubereitung mit einem Treibgas aufgeschäumt ist. Das Treibgas wird dabei erfindungsgemäß vorteilhaft in einer Menge von 0 bis 20 Gewichts-%, besonders vorteilhaft in einer Menge von 5 bis 15 und ganz besonders vorteilhaft in einer Menge von 8 bis 11 Gewichts-%, jeweils bezogen auf das Gesamtgewicht der Formulierung eingesetzt. Die Verwendung einer Pumpschäumvorrichtung für treibmittelfreie Schaumfestiger ist ebenfalls möglich.

Zum Aufschäumen der Zubereitung werden dabei erfindungsgemäß bevorzugt Propan/Butan-Gemische eingesetzt.

Natürlich weiß der Fachmann, dass es an sich nichttoxische Treibgase gibt, die grundsätzlieh für die Verwirklichung der vorliegenden Erfindung in Form von Aerosolpräparaten geeignet wären, auf die aber dennoch wegen bedenklicher Wirkung auf die Umwelt öder sonstiger Begleitumstände verzichtet werden sollte, insbesondere Fluorkohlenwasserstoffe und Fluorchlorkohlenwasserstoffe (FCKW).

Eine besonders bevorzugte Ausführungsform der erfindungsgemäßen Zubereitung ist ihr Vorliegen in Form einer wässrigen oder wässrig-alkoholischen Lösung.

Erfindungsgemäß vorteilhaft enthält die erfindungsgemäße Zubereitung weitere kosmetische Hilfsstoffe z.B. Pflegestoffe, Konservierungsmittel, Bakterizide, Parfüme, Emulgatoren. weichmachende, anfeuchtende und/oder feuchthaltende Substanzen, Fette, Öle, Wachse, Fettalkohole, Fettsäureester oder andere übliche Bestandteile einer kosmetischen oder dermatologischen Formulierung wie Alkohole, Polyole, Polymere, Schaumstabilisatoren, Elektrolyte, organische Lösungsmittel oder Siliconderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Erfindungsgemäß vorteilhaft enthält die erfindungsgemäße Zubereitung 0,1 bis 0,4 Gewichts-%, bezogen auf das Gesamtgewicht der Zubereitung, Zitronensäure und/oder deren Salze.

Vorteilhaft im Sinne der vorliegenden Erfindung ist es der erfindungsgemäßen Zubereitung Panthenol zuzusetzen. Der Gehalt an Panthenol beträgt dabei erfindungsgemäß vorteilhaft 0,01 bis 0,3 Gewichts-%, bezogen auf das Gesamtgewicht der Zubereitung.

Erfindungsgemäß vorteilhaft enthält die erfindungsgemäße Zubereitung einen oder mehrere O/W-Emulgatoren. Dabei ist insbesondere der Einsatz von Polyethylene Glycol 2000 Hydrogenated Castor Oil (INCI: PEG-40 Hydrogenated Castor Oil) erfindungsgemäß besonders bevorzugt. Erfindungsgemäß vorteilhaft hat sich dabei eine Einsatzkonzentration von 0,05 bis 0,5 Gewichts-%, bezogen auf das Gesamtgewicht der Zubereitung, erwiesen.

Erfindungsgemäß enthält die erfindungsgemäße Zubereitung Cetyltrimethylammoniumchlorid (Cetrimonium chloride; CAS 112-02-7), welches bevorzugt in einer Konzentration von 0,1 bis 2 Gewichts-%, bezogen auf das Gesamtgewicht der Zubereitung in dieser enthalten ist.

Erfindungsgemäß bevorzugt ist die Verwendung der kosmetische Zubereitung als Haarfestiger. Erfindungsgemäß besonders bevorzugt ist die Verwendung der erfindungsgemäßen Zubereitung als Schaumfestiger für die Frisurgestaltung.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Rezepturbeispiele:**

| | **1** | **2** | **3** | **4** | **5** |
|---|---|---|---|---|---|
| Copolymer aus N-Vinylpyrrolidon (VP), N-Vinylimidazol (VI), Methacrylamid (MAM) und quaternisiertem N-Vinylimidazol (QVI) | 7 | 5 | 5 | 7 | 5 |
| VP/VA Copolymer | 5 | 7 | 9 | - | - |
| VP/Methacrylamide / Vinyl Imidazole Copolymer | - | - | - | 10 | 15 |
| Polyquaternium-16 | - | - | - | 0,5 | - |
| polyquaternium-4 | - | - | 0,5 | - | - |
| Citronensäure | 0,2 | 0,2 | 0,3 | 0,2 | 0,3 |
| Cetrimoniumchlorid | 1,0 | 1,0 | 1,0 | 1,0 | 1,0 |
| Hydroxyethyl Cetyldimonium Phosphate | 0,5 | 0,5 | 0,5 | 0,5 | 0,5 |
| Dimethicone Copolyol | - | - | - | 0,2 | - |
| Panthenol | 0,1 | 0,1 | 0,1 | 0,1 | 0,1 |
| Natriumbenzoat | 0.2 | 0,2 | - | - | 0,2 |
| PEG-40 Hydrogenated Castor Oil | 0,2 | 0,2 | 0,2 | 0,2 | 0,2 |
| Parfüm | q.s. | q.s. | q.s. | q.s. | q.s. |
| Propan/Butan | 10 | 10 | 10 | 10 | 10 |
| Ethanol | - | - | 15 | 15 | - |
| Wasser | ad 100 | ad 100 | ad 100 | ad 100 | ad 100 |

## Patentansprüche

1. Kosmetische Zubereitung enthaltend eine Kombination aus
a) einem Copolymer gebildet aus den Monomeren N-Vinylpyrrolidon (VP), N-Vinylimidazol (VI), Methacrylamid (MAM) und quaternisiertem N-Vinylimidazol (QVI),
b) mindestens einem nichtionischen Polymer, wobei ein oder mehrere nichtionische Polymere gewählt werden aus der Gruppe der Homopolymere des Vinylpyrrolidons, des Vinylcaprolactams, aus der Gruppe der Copolymerisate aus Vinylpyrrolidon und Vinylacetat, aus der Gruppe der Polyvinylcaprolactame, Polyvinylamide, aus der Gruppe der Copolymerisate aus Vinylpyrrolidon und Dimethylaminoethylmethacrylat, aus der Gruppe der Terpolymere aus Vinylpyrrolidon, Vinylimidazol und Methacrylamid oder aus der Gruppe der Terpolymere aus Vinylpyrrolidon, Vinylimidazol und Vinylcaprolactam,
c) Cetyltrimethylammoniumchlorid (Cetrimonium chloride; CAS 112-02-7).

2. Kosmetische Zubereitung nach Anspruch 1, **dadurch gekennzeichnet, dass** die Zubereitung das Copolymer a) in einer Konzentration von 0,01 bis 10 Gewichts-%, bezogen auf das Gesamtgewicht der Zubereitung, enthält.

3. Kosmetische Zubereitung nach einem der Ansprüche 1 oder 2, **dadurch gekennzeichnet, dass** die Zubereitung nichtionische Polymere in einer Konzentration von 0,01 bis 20 Gewichts-%, bezogen auf das Gesamtgewicht der Zubereitung, enthält.

4. Kosmetische Zubereitung nach einem der vorhergehenden Ansprüche, **dadurch gekennzeichnet, dass** das Gewichtsverhältnis von Copolymer a) zu nichtionischen Polymeren von 5:1 bis 1:5 beträgt.

5. Kosmetische Zubereitung nach einem der vorhergehenden Ansprüche, **dadurch gekennzeichnet, dass** das Copolymer a) die Monomere N-Vinylpyrrolidon (VP), N-Vinylimidazol (VI), Methacrylamid (MAM) und quaternisiertem N-Vinylimidazol (QVI) in einem Gewichtsverhältnis von 55:10:29:6 enthält.

6. Kosmetische Zubereitung nach einem der vorhergehenden Ansprüche, **dadurch gekennzeichnet, dass** als nichtionisches Polymer ein Copolymerisat aus Vinylpyrrolidon und Vinylacetat (VP/VA-Copolymer) eingesetzt wird

7. Kosmetische Zubereitung nach einem der vorhergehenden Ansprüche, **dadurch gekennzeichnet, dass** die Zubereitung mit einem Treibgas aufgeschäumt sein kann.

8. Verwendung einer kosmetischen Zubereitung nach einem der vorhergehenden Ansprüche als Haarfestiger, insbesondere als Schaumfestiger.

## Claims

1. Cosmetic preparation comprising a combination of
a) a copolymer formed from the monomers N-vinylpyrrolidone (VP), N-vinylimidazole (VI), methacrylamide (MAM) and quaternized N-vinylimidazole (QVI),
b) at least one nonionic polymer, where one or more nonionic polymers are selected from the group of the homopolymers of vinylpyrrolidone, of vinylcaprolactam, from the group of the copolymers of vinylpyrrolidone and vinyl acetate, from the group of the polyvinylcaprolactams, polyvinylamides, from the group of the copolymers of vinylpyrrolidone and dimethylaminoethyl methacrylate, from the group of the terpolymers of vinylpyrrolidone, vinylimidazole and methacrylamide or from the group of the terpolymers of vinylpyrrolidone, vinylimidazole and vinylcaprolactam,
c) cetyltrimethylammonium chloride (Cetrimonium chloride; CAS 112-02-7).

2. Cosmetic preparation according to Claim 1, **characterized in that** the preparation comprises the copolymer a) in a concentration of from 0.01 to 10% by weight, based on the total weight of the preparation.

3. Cosmetic preparation according to one of Claims 1 or 2, **characterized in that** the preparation comprises nonionic polymers in a concentration of from 0.01 to 20% by weight, based on the total weight of the preparation.

4. Cosmetic preparation according to one of the preceding claims, **characterized in that** the weight ratio of copolymer a) to nonionic polymers is from 5:1 to 1:5.

5. Cosmetic preparation according to one of the preceding claims, **characterized in that** the copolymer a) comprises the monomers N-vinylpyrrolidone (VP), N-vinylimidazole (VI), methacrylamide (MAM) and quaternized N-vinylimidazole (QVI) in a weight ratio of 55:10:29:6.

6. Cosmetic preparation according to one of the preceding claims, **characterized in that** a copolymer of vinylpyrrolidone and vinyl acetate (VP/VA copolymer) is used as nonionic polymer.

7. Cosmetic preparation according to one of the preceding claims, **characterized in that** the preparation can be foamed using a propellant gas.

8. Use of a cosmetic preparation according to one of the preceding claims as hair setting composition, in particular as foam setting composition.

## Revendications

1. Préparation cosmétique contenant une combinaison de
a) un copolymère formé par les monomères N-vinylpyrrolidone (VP), N-vinylimidazole (VI), méthacrylamide (MAM) et N-vinylimidazole quaternisé (QVI),
b) au moins un polymère non ionique, un ou plusieurs polymères non ioniques étant choisis dans le groupe des homopolymères de vinylpyrrolidone, de vinylcaprolactame, dans le groupe des copolymères de vinylpyrrolidone et d'acétate de vinyle, dans le groupe des polyvinylcaprolactames, des polyvinylamides, dans le groupe des copolymères de vinylpyrrolidone et de méthacrylate de diméthylaminoéthyle, dans le groupe des terpolymères de vinylpyrrolidone, de vinylimidazole et de méthacrylamide, ou dans le groupe des terpolymères de vinylpyrrolidone, de vinylimidazole et de vinylcaprolactame,
c) du chlorure de cétyltriméthylammonium (chlorure de cétrimonium ; CAS 112-02-7).

2. Préparation cosmétique selon la revendication 1, **caractérisée en ce que** la préparation contient le copolymère a) en une concentration de 0,01 à 10 % en poids, par rapport au poids total de la préparation.

3. Préparation cosmétique selon l'une quelconque des revendications 1 ou 2, **caractérisée en ce que** la préparation contient des polymères non ioniques en une concentration de 0,01 à 20 % en poids, par rapport au poids total de la préparation.

4. Préparation cosmétique selon l'une quelconque des revendications précédentes, **caractérisée en ce que** le rapport en poids entre le copolymère a) et les polymères non ioniques est de 5:1 à 1:5.

5. Préparation cosmétique selon l'une quelconque des revendications précédentes, **caractérisée en ce que** le copolymère a) contient les monomères N-vinylpyrrolidone (VP), N-vinylimidazole (VI), méthacrylamide (MAM) et N-vinylimidazole quaternisé (QVI) en un rapport en poids de 55:10:29:6.

6. Préparation cosmétique selon l'une quelconque des revendications précédentes, **caractérisée en ce qu'**un copolymère de vinylpyrrolidone et d'acétate de vinyle (copolymère VP/VA) est utilisé en tant que polymère non ionique.

7. Préparation cosmétique selon l'une quelconque des revendications précédentes, **caractérisée en ce que** la préparation peut être moussée avec un gaz gonflant.

8. Utilisation d'une préparation cosmétique selon l'une quelconque des revendications précédentes en tant que laque pour cheveux, notamment laque en mousse.
